# EUROPÄISCHE PATENTANMELDUNG

(11) **EP 2 853 533 A1**
(43) Veröffentlichungstag der Anmeldung: **01.04.2015**
(21) Anmeldenummer: 13186560.2
(22) Anmeldetag: 29.09.2013
(51) Int. Cl.: C07D 471/04, A61K 31/437

(54) **6-Fluor-9-methyl-ß-carbolin**

(71) Anmelder: Audiocure Pharma GmbH, 14193 Berlin (DE)
(72) Erfinder: Rommelspacher, Hans, 14193 Berlin (DE); Enzensperger, Christoph, 07745 Jena (DE)
(74) Vertreter: Arth, Hans-Lothar

(57) **Zusammenfassung**

Die vorliegende Erfindung betrifft das ß-Carbolin 6-Fluor-9-methyl-β-carbolin, dessen Herstellung sowie dessen Verwendung in pharmazeutischen Zusammensetzungen.

## Beschreibung

Die vorliegende Erfindung betrifft das ß-Carbolin 6-Fluor-9-methyl-ß-carbolin, dessen Herstellung sowie dessen medizinische Verwendung und pharmazeutische Zusammensetzungen enthaltend 6-Fluor-9-methyl-ß-carbolin.

Die erfindungsgemäße Verbindung dient zur Behandlung von akuten und chronischen Ohrenerkrankungen und Hörschäden, Schwindel und Gleichgewichtsstörungen insbesondere von Hörsturz, Knalltrauma, Explosionstrauma, Innenohrschwerhörigkeit durch chronische Lärmexposition, Altersschwerhörigkeit, Trauma während der Implantation von Innenohrprothesen (Insertionstrauma), Schwindel aufgrund von Erkrankungen im Innenohrbereich, Schwindel im Zusammenhang und/oder als Symptom der Menière'schen Erkrankung, Gleichgewichtsstörungen im Zusammenhang und/oder als Symptom der Menière'schen Erkrankung, Tinnitus und Hörschäden aufgrund von Antibiotika und Zytostatika.

Nach Erhebungen der Weltgesundheitsorganisation (WHO) leiden ca. 250 Millionen Menschen weltweit an leichten oder schweren Hörstörungen. In den USA sind 30 bis 40 Millionen Menschen von Hörschädigungen und von Hörverlusten betroffen. Allein hier betragen die Kosten der Behandlungen ca. 50 Milliarden USD jährlich. Der deutsche Schwerhörigenbund berichtete im Jahr 2007, dass ca. 19 % der deutschen Bevölkerung über 14 Jahre an Hörstörungen leiden.

Mit zunehmendem Alter nimmt der Prozentsatz an Hörgeschädigten zu. Bereits jetzt stellen die Hörstörungen bei über 65-jährigen Menschen die vierthäufigste chronische körperliche Beeinträchtigung, nach Erkrankungen der Knochen und Gelenke, Bluthochdruck und Herzkrankheiten, dar. Unter den 60 bis 69-jährigen sind 37 %, unter den 70-jährigen und älteren über 54 % der Bevölkerung von Hörschädigungen betroffen. In der Bundesrepublik Deutschland leiden ca. 12-15 Millionen Patienten an Innenohrschwerhörigkeit und ca. 2,9 Millionen Patienten an Tinnitus.

Der Begriff "Tinnitus aurium" oder kurz Tinnitus bezeichnet ein Symptom oder auch Syndrom, bei dem der Betroffene anhaltende oder wiederkehrende Geräusche wahrnimmt, wobei zwei Formen des Tinnitus unterschieden werden können. Beim relativ seltenen "objektiven Tinnitus" sind die vom Patienten wahrgenommenen Geräusche auch von außen wahrzunehmen oder zumindest zu messen. Sie beruhen in der Regel auf einer körpereigenen Schallquelle wie z.B. auf einem Blutgefäß, das nah am Innenohr vorbeiläuft. Beim deutlich häufigeren "subjektiven Tinnitus" beruhen die akustischen Phänomene hingegen nicht auf einer äußeren für andere Personen wahrnehmbaren Quelle. Damit ist der "subjektive Tinnitus" eine akustische Wahrnehmung, die unabhängig von einem Schall, der auf das Ohr wirkt, vom Patienten wahrgenommen wird. Die Ursachen für diese Wahrnehmung können vielfältig sein und unter anderem auf eine Störung der Hörfunktion des Innenohrs zurückzuführen sein. Man sollte jedoch den Tinnitus deutlich von akustischen Halluzinationen abgrenzen. Die Art der scheinbaren Geräusche, welche der Tinnitus-Patient wahrnimmt, ist sehr vielfältig. Man fasst unter anderem folgende akustische Eindrücke unter dem Begriff Tinnitus zusammen:
- Brumm- oder Pfeiftöne
- Zischen
- Rauschen
- Knacken oder Klopfen

Das Geräusch kann in seiner Intensität gleichbleibend sein, es kann jedoch auch einen rhythmisch-pulsierenden Charakter haben.

Das Ohr ist folgendermaßen aufgebaut. Von der Ohrmuschel aus führt der Gehörgang in das Innere des Ohrs. Der Gehörgang endet am Trommelfell. Diesen Teil des Ohrs bezeichnet man als Äußeres Ohr. Hinter dem Äußeren Ohr liegt ein Raum, das Mittelohr, auch Tympanon genannt, welches mit der Eustachischen Röhre und damit auch mit dem Nasopharynx verbunden ist. Das Mittelohr wird auf seiner einen Seite vom Trommelfell und auf der gegenüberliegenden Seite durch das Ovale Fenster und durch das Runde Fenster abgeschlossen. Hinter den beiden Fenstern schließt sich der als Innenohr bezeichnete Raum an. Das Innenohr oder auch das knöcherne Labyrinth beherbergt mehrere Organe des Ohrs wie das Gleichgewichtsorgan (Vestibularapparat) und die so genannte Schnecke (Cochlea) für die akustische Wahrnehmung. Diese wiederum enthält das Cortische Organ als Träger der Sensorzellen (Haarzellen) im Innenohr.

Die Membran des Runden Fensters bildet eine biologische Barriere zum Innenohrraum und stellt das größte Hindernis für die lokale Therapie von Schädigungen und Erkrankungen des Innenohres dar. Der verabreichte Wirkstoff muss diese Membran überwinden, um in den Innenohrraum zu gelangen. Der Wirkstoff kann aber nicht mechanisch-apparativ durch die Membran appliziert werden, da sonst die Membran durch diese Manipulation geschädigt wird. Er kann jedoch operativ (z.B. Injektion durch das Trommelfell) an der Rundfenstermembran lokal appliziert werden und dann die Rundfenstermembran penetrieren. So gelangt der Wirkstoff in den mit Perilymphe gefüllten Innenohrraum,der durch die miteinander verbundenen Kompartimente der Cochlea, nämlich der Scala tympani und der Scala vestibuli, gebildet wird. In einem durch Membranen abgetrennten Kompartiment zwischen der Scala tympani und der Scala vestibuli, der als Scala media (auch Ductus cochlearis) bezeichnet wird, befinden sich von Endolymphe umgeben die Sinneszellen des Hörorgans (innere und äußere Haarzellen). Diese werden zusammen mit den umliegenden Stützzellen als Cortisches Organ bezeichnet. Die Haarzellen sind bei Innenohrschwerhörigkeit jeglicher Ursache (Alter, Medikamente wie beispielsweise bestimmte Antibiotika und Zytostatika) sowie bei Tinnitus primär geschädigt. Über die Membranen, die die Scala media vom perilymphatischen Raum abtrennen, können Wirkstoffe auch in die Endolymphe und so oder direkt zu den Haarzellen gelangen. Da sowohl die perilymphatischen als auch die endolymphatischen Kompartimente der Cochlea mit denen des Gleichgewichtsorgans in Verbindung stehen, können Wirkstoffe von der Cochlea auch an die Sinneszellen des Vestibularapparates gelangen.

Die deutsche Patentanmeldung (DE 10 2007 009264 A1) offenbart 9-Alkyl-ß-carboline, die aufgrund ihrer neuroprotektiven Wirkung zur Therapie und/oder Prophylaxe von Bewegungsstörungen und/oder neurodegenerativen Krankheiten wie z.B. Alzheimer oder Parkinson angewendet werden können.

Von Hamann et al. (J. Hamann et al.; Neurochem Internat. 2008, 52, 688-700) konnte eine neuroprotektive Wirkung des ß-Carbolins 9-Methyl-ß-carbolin an primären Zellkulturen mesencephaler Neurone gezeigt werden. Die dort gezeigte erhöhte Überlebensrate der Neurone durch 9-Methyl-ß-carbolin war jedoch nicht auf eine erhöhte Proliferation zurückzuführen. Im Gegenteil, die Applikation von 9-Methyl-ß-carbolin führte zu einer Hemmung der Proliferation und zu einer gesteigerten Differenzierung der Neurone. Diese Wirkung von 9-Methyl-ß-carbolin konnte an humanen Neuroblastom-Zellen (SH-SY5Y) bestätigt werden. Einhergehend mit der geförderten Differenzierung konnte eine gesteigerte Expression von neurotrophen Faktoren wie sonic hedgehog, Wnt1 und Wnt5a nachgewiesen werden. Zudem zeigte sich in der primären Zellkultur mesencephaler Neurone eine 9-Methyl-ß-carbolin-induzierte Erhöhung der Zahl an dopaminergen Neuronen. Daher wird ein therapeutischer Nutzen von 9-Methyl-ß-carbolin bei der Behandlung von Parkinson in Aussicht gestellt.

Die US-Patentanmeldung (US 2004/038970 A1) offenbart die Verwendung von ß-Carbolinen als Wirkstoff zur Behandlung einer Vielzahl medizinischer Indikationen, einschließlich Tinnitus, wobei jedoch eine strukturell stark von 9-Alkyl-ß-carbolinen und somit auch von 6-Fluor-9-methyl-β-carbolin abweichende Gruppe an ß-Carbolinen genannt wird.

Die internationale Patentanmeldung (WO 2011/079841) offenbart ß-Carboline, insbesondere 9-Alkyl-ß-carboline und deren Verwendung zur Behandlung von Erkrankungen bzw. Schädigungen des Innenohrs. Es konnte gezeigt werden, dass die Applikation von 9-Alkyl-ß-carbolinen die neuronale Differenzierung von humanen Neuroblastom-Zellen (SH-SY5Y) sowie die Expression von neurotrophen Faktoren mit neuroprotektiven Eigenschaften fördert. Dies konnte in Zellkulturen von Cochlea-Präparationen der Ratte bestätigt werden. Zusätzlich konnte am Meerschweinchen gezeigt werden, dass 9-Methyl-ß-carbolin nach lokaler Applikation am Runden Fenster des Innenohrs in den perilymphatischen Raum des Innenohrs diffundieren kann und zu einer Erhöhung der elektrophysiologischen Aktivität der Haarsinneszellen des Cortischen Organs führt.

Die erfindungsgemäße Verbindung 6-Fluor-9-methyl-ß-carbolin ist im Stand der Technik nicht in individualisierter, d.h. konkreter Form offenbart. Zudem hat sich überraschenderweise gezeigt, dass sich das erfindungsgemäße 6-Fluor-9-methyl-ß-carbolin durch besondere Eigenschaften vom Stand der Technik abhebt, die eine therapeutische Verwendung begünstigen. So konnte für das erfindungsgemäße 6-Fluor-9-methyl-ß-carbolin eine höhere Wirksamkeit im Vergleich zu 9-Methyl-ß-carbolin bei der Förderung der neuronalen Differenzierung in humanen Neuroblastom-Zellen nachgewiesen werden (siehe Beispiel 2). Zudem zeigt 6-Fluor-9-methyl-ß-carbolin im Vergleich zu 9-Methyl-ß-carbolin eine deutlich erhöhte Lipophilie, was mit einer erhöhten Membranpermeabilität einhergeht. Diese hat weitreichende Konsequenzen für die therapeutische Anwendung der erfindungsgemäßen Verbindung zur Behandlung von Erkrankungen bzw. Schädigungen des Innenohres. Wie bereits beschrieben bilden biologische Membranen wie das Trommelfell, die Membran des Runden Fensters und die Membranen der Scala media (Membrana tectoriana und Reißner Membran) ein natürliches Hindernis für einen Wirkstoff auf dem Weg zu den Haarzellen des Innenohres. Dies gilt insbesondere bei der topischen Applikation eines Wirkstoffes auf dem Trommelfell oder dem Runden Fenster. Durch eine erhöhte Membranpermeabilität wird daher die Verfügbarkeit des Wirkstoffes an den Zielzellen erhöht. Neben der Membranpermeabilität spielt auch der Abbau eines Wirkstoffs im Körper eine entscheidende Rolle für dessen Wirksamkeit, hier insbesondere bei systemischer Verabreichung und auch für eventuelle Nebenwirkungen. Während das 9-Methyl-ß-carbolin über eine Hydroxylierung an Position 6 mit anschließender Konjugationsreaktion abgebaut wird, ist dieser Metabolisierungsweg beim 6-Fluor-9-methyl-ß-carbolin durch die Fluoridgruppe blockiert. Hierdurch konnte nicht nur die Halbwertszeit des 6-Fluor-9-methyl-ß-carbolins erhöht, sondern zudem das Risiko für die Bildung potentiell toxischer Abbauprodukte verringert werden. Aufgrund der erhöhten Wirksamkeit des 6-Fluor-9-methyl-ß-carbolins erscheint eine im Vergleich zu anderen 9-Alkyl-ß-carbolinen geringere Dosierung bei der Therapie möglich. Dies ist gerade für eine Langzeittherapie bei chronischen Erkrankungen von enormer Bedeutung, da hierdurch die Belastung des Körpers und zudem unerwünschte Nebenwirkungen verringert werden können. Es handelt sich folglich bei der vorliegenden Anmeldung um eine Auswahlerfindung.

Der vorliegenden Erfindung liegt die Aufgabe zugrunde, einen Wirkstoff sowie pharmazeutische Formulierungen bereitzustellen, welche zur Prophylaxe und Behandlung von Hörschäden, Altersschwerhörigkeit, Schwindel und Gleichgewichtsstörungen eingesetzt werden können und einen Vorteil zum Stand der Technik aufweisen.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen, der Beschreibung und den Beispielen.

Die vorliegende Erfindung betrifft die Verbindung 6-Fluor-9-methyl-β-carbolin der Formel (I) 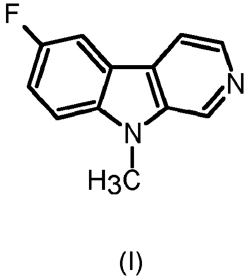 sowie pharmakologisch verträgliche Salze, Solvate und Hydrate sowie Prodrugs und Komplexverbindungen der vorgenannten Verbindung.

Der hierin verwendete Ausdruck Prodrug ist definiert als eine pharmakologische Substanz, die in einer inaktiven oder weniger wirksamen Form verabreicht wird. Nach Gabe wird sie dann im Körper des Patienten in ihre aktive, wirksame Form, 6-Fluor-9-methyl-ß-carbolin, umgewandelt.

Als mögliche Säuren, welche ein Säureadditionssalz mit der Verbindung der vorliegenden Erfindung bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure. Zudem sind auch Betain-Formen möglich.

Ein weiterer Aspekt der vorliegenden Erfindung betrifft ein Verfahren zur Herstellung der erfindungsgemäßen Verbindung, das die folgenden Schritte umfasst:
a) Reaktion der Ausgangsverbindung 5-Fluor-1-methyltryptaminhydrochlorid der Formel (II) unter Zugabe von Glyoxalsäurehydrat der Formel (III) und einer Base zur Verbindung der Formel (IV) 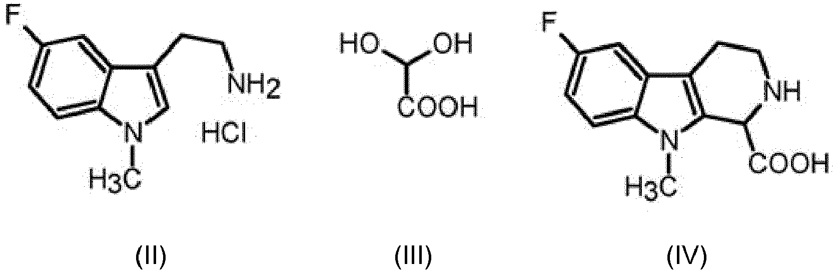
b) Decarboxylierung der Verbindung der Formel (IV) unter Zugabe einer Säure und unter Erhitzen zu 6-Fluor-9-methyl-2,3,4,9-tetrahydro-ß-carbolin der Formel (V) 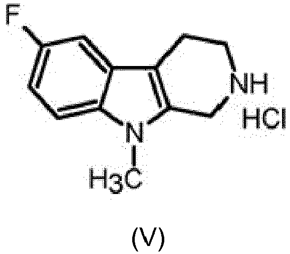
c) Aromatisierung von 6-Fluor-9-methyl-2,3,4,9-tetrahydro-ß-carbolin (V) zu 6-Fluor-9-methyl-β-carbolin (I) unter Zugabe eines Katalysators.

Das Ausgangsprodukt 5-Fluor-1-methyltryptaminhydrochlorid ist kommerziell erhältlich, z.B. über die AKos GmbH (Steinen). In einer bevorzugten Ausführungsform der Erfindung wird durch Zugabe der Base in Schritt a) ein pH-Wert von zwischen 2 und 6, bevorzugt zwischen 3 und 5 und besonders bevorzugt ein pH-Wert von 4 erreicht. In einer weiteren bevorzugten Ausführungsform wird die Kristallisation der Verbindung IV in einem Schritt a)' durch eine Inkubation in Eiswasser gefördert.

In einer weiteren bevorzugten Ausführungsform erfolgt die Reaktion in Schritt b) unter Inertgas, besonders bevorzugt unter N₂. Des Weiteren wird bevorzugt, die Kristallisation des Reaktionsproduktes aus Schritt b) durch einen Schritt b)' Inkubation bei Kälte, besonders bevorzugt zwischen 0°C und 5°C, über mehrere Stunden zu fördern. In einer weiteren bevorzugten Ausführungsform wird zur Aufreinigung des Reaktionsproduktes aus Schritt b) nach der Kristallisation eine Umkristallisation aus Methanol durchgeführt. Des Weiteren wird bevorzugt, das 6-Fluor-9-methyl-2,3,4,9-tetrahydro-ß-carbolin in Schritt c) zunächst in Wasser zu lösen und durch Zugabe einer Base, z.B. Natriumhydroxid oder Natriumcarbonat, auf einen basischen pH-Wert einzustellen, besonders bevorzugt einen pH-Wert zwischen pH 11 und pH 14, besonders bevorzugt pH 13.

In einer bevorzugten Ausführungsform, ist als Katalysator für die oxidative Aromatisierung in Schritt c) unter anderem Pd/C, Pt/C, PdCl₂, Pd / Sepiolith, Pd/SiO₂ und Pd/AlO₄ möglich. Dem Fachmann sind zudem weitere Zusätze bekannt, die eine oxidative Aromatisierung ermöglichen. Bevorzugt wird jedoch Pd/C (*Palladium on carbon,* Palladium auf Aktivkohle) als Katalysator eingesetzt. Bei der Zugabe einer Base in Schritt a) und Schritt c) kann es sich unabhängig voneinander um anorganische oder organische Basen wie z.B. NaOH, KOH, NH₄OH, Tetraalkylammonium Hydroxid, Lysin oder Arginin handeln. Dem Fachmann ist bekannt, welche Basen sich hierbei zur Durchführung der Reaktion eignen. Bevorzugt sind jedoch KOH bzw. NaOH.

Bei der Zugabe einer Säure in Schritt b), kann es sich um anorganische und organische Säuren wie z.B. Salzsäure, Bromwasserstoffsäure, Schwefelsäure, Phosphorsäure, Essigsäure, Zitronensäure, Oxalsäure, Äpfelsäure, Salicylsäure, p-Aminosalicylsäure, Malonsäure, Fumarsäure, Bernsteinsäure, Ascorbinsäure, Maleinsäure, Sulfonsäure, Phosphonsäure, Perchlorsäure, Salpetersäure, Ameisensäure, Propionsäure, Gluconsäure, Milchsäure, Weinsäure, Hydroxymaleinsäure, Brenztraubensäure, Phenylessigsäure, Benzoesäure, p-Aminobenzoesäure, p-Hydroxybenzoesäure, Methansulfonsäure, Ethansulfonsäure, salpetrige Säure, Hydroxyethansulfonsäure, Ethylensulfonsäure, p-Toluolsulfonsäure, Naphthylsulfonsäure, Sulfanilsäure, Camphersulfonsäure, Chinasäure, Mandelsäure, o-Methylmandelsäure, Hydrogenbenzolsulfonsäure, Pikrinsäure, Adipinsäure, d-o-Tolylweinsäure, Tartronsäure, α-Toluylsäure, (o-, m-, p-) Toluylsäure, Naphthylaminsulfonsäure handeln. Dem Fachmann ist bekannt, welche Säure sich hierbei zur Durchführung der Reaktion eignet. Bevorzugt ist jedoch Salzsäure.

Es konnte überraschenderweise gezeigt werden, dass die erfindungsgemäße Verbindung 6-Fluor-9-methyl-ß-carbolin (I) im Vergleich zu bereits bekannten und getesteten ß-Carbolinen, wie 9-Methyl-ß-Carbolin eine gesteigerte Wirksamkeit bei der Behandlung von Innenohrerkrankungen aufweist. Zusätzlich zeigen mehrere Experimente eine deutliche erhöhte Lipophilie der erfindungsgemäßen Verbindung. Gegenüber der Vergleichssubstanz 9-Methyl-ß-carbolin ergeben sich u.a. die folgenden Vorteile:
1. Die Membrangängigkeit ist erhöht.
2. Die Elimination ist verlangsamt (HWZ von 9-Methyl-ß-carbolin ca. 60 Minuten).
3. Da von anderen ß-Carbolinen bekannt ist, dass sie durch Hydroxylierung an Position 6 und eine anschließende Konjugationsreaktion abgebaut werden, ist durch eine Blockierung dieses Metabolisierungsweges durch die Fluoridgruppe an Position 6.
   - Die Bioverfügbarkeit nach oraler Applikation ist verbessert.
   - Das Risiko von toxischen Metaboliten oder potentiell allergenen Metaboliten minimiert.

Die erfindungsgemäße Verbindung ist besonders geeignet zur Behandlung von Erkrankungen bzw. Schädigungen des Innenohrs, insbesondere der neuronalen und sensorischen Strukturen des Innenohres. Diese Erkrankungen bzw. Schädigungen betreffen hierbei bevorzugt das Gehör und/oder die Vestibularfunktion. Begleitende Symptome umfassen unter anderem Hörschäden, Hörverlust, Schwindel, Gleichgewichtsstörungen, Tinnitus, Orientierungslosigkeit, Übelkeit und Erbrechen.

Somit betrifft ein Aspekt der vorliegenden Erfindung die Verbindung 6-Fluor-9-methyl-β-carbolin zur Verwendung als Medikament. Die erfindungsgemäße Verbindung ist besonders geeignet zur Behandlung von Erkrankungen und/oder Schädigungen des Innenohres, insbesondere Innenohrschwerhörigkeit. Bei den Erkrankungen und/oder Schädigungen des Innenohres handelt es sich dabei bevorzugt um akute und chronische Erkrankungen des Innenohres. Zu den akuten Erkrankungen des Innenohres zählen unter anderem Knalltrauma, Explosionstrauma des Ohres, Hörsturz und Trauma während der Implantation von Innenohrprothesen (Insertionstrauma). Die chronischen Erkrankungen des Innenohres umfassen unter anderem chronisches Schalltrauma (Innenohrschwerhörigkeit durch chronische Lärmexposition), Presbyakusis (Altersschwerhörigkeit), Otosklerose und die Menière'sche Erkrankung. Der Begriff Innenohrschwerhörigkeit (auch als cochleäre bzw. sensorische Schwerhörigkeit bezeichnet) umfasst sowohl die Schwerhörigkeit durch chronische Lärmexposition (auch als chronische Lärmschwerhörigkeit oder chronisches Lärmtrauma bezeichnet) als auch die Presbyakusis (Altersschwerhörigkeit).

Erkrankungen und Schädigungen des Innenohrs können zudem durch ototoxische Substanzen wie Antibiotika und/oder Zytostatika ausgelöst werden. Die erfindungsgemäße Verbindung ist geeignet zur Behandlung von Erkrankungen und/oder Schädigungen des Innenohres, die dadurch gekennzeichnet sind, dass die Schädigung des Innenohrs durch ototoxische Substanzen bedingt ist. Bei den ototoxischen Substanzen handelt es sich dabei bevorzugt um Antibiotika und/oder Zytostatika, besonders bevorzugt aus der Gruppe umfassend oder bestehend aus: Ampicillin, Bacampicillin, Carbenicillin, Indanyl, Mezlocillin, Piperacillin, Ticarcillin, Amoxicillin-Clavulansäure, Ampicillin-Sulbactam, Benzylpenicillin, Cloxacillin, Dicloxacillin, Methicillin, Oxacillin, Penicillin G, Penicillin V, Piperacillin, Tazobactam, Ticarcillin, Clavulansäure, Nafcillin, Cephalosporin, Cefadroxil, Cefazolin, Cephalexin, Cephalothin, Cephapirin, Cephradin, Cefaclor, Cefamandol, Cefonicid, Cefotetan, Cefoxitin, Cefprozil, Ceftmetazol, Cefuroxim, loracarbef, Cefdinir, Ceftibuten, Cefoperazon, Cefixim, Cefotaxim, Cefpodoxim Proxetil, Ceftazidim, Ceftizoxim, Ceftriaxon, Cefepim, Azithromycin, Clarithromycin, Clindamycin, Dirithromycin, Erythromycin, Roxithromycin, Telithromycin, Cethromycin, Spiramycin, Ansymacin, Oelandomycin, Lincomycin, Troleandomycin, Cinoxacin, Ciprofloxacin, Enoxacin, Gatifloxacin, Grepafloxacin, Levofloxacin, lomefloxacin, Moxifloxacin, Nalidixinsäure, Norfloxacin, Ofloxacin, Sparfloxacin, Trovafloxacin, Oxolininsäure, Gemifloxacin, Perfloxacin, Imipenem-Cilastatin, Meropenem, Aztreonam, Amikacin, Gentamicin, Kanamycin, Neomycin, Netilmicin, Streptomycin, Tobramycin, Paromomycin, Teicoplanin, Vancomycin, Demeclocyclin, Doxycyclin, Methacyclin, Minocyclin, Oxytetracyclin, Tetracyclin, Chlortetracyclin, Tigecyclin, Mafenid, Silbersulfadiazin, Sulfacetamid, Sulfadiazin, Sulfamethoxazol, Sulfasalazin, Sulfisoxazole, Trimethoprim-Sulfamethoxazol, Sulfamethizol, Rifabutin, Rifampin, Rifapentin, Linezolid, Streptogramins, Quinopristin, Dalfopristin, Bacitracin, Chloramphenicol, Fosfomycin, Isoniazid, Methenamin, Metronidazol, Mupirocin, Nitrofurantoin, Nitrofurazon, Novobiocin, Polymyxin, Spectinomycin, Trimethoprim, Colistin, Cycloserin, Capreomycin, Ethionamide, Pyrazinamide, Para-Aminosalicyclsäure, Erythromycin-2-Acetat, Erythromycin-2-Stearat, Erythromycinestolat, Erythromycinethylsuccinat, Erythromycinglutamat, Rifampicin, Miconazol, Ketoconazol, Clotrimazol, Econazol, Bifonazol, Butoconazol, Fenticonazol, Isoconazol, Oxiconazol, Sertaconazol, Sulconazol, Tioconazol, Fluconazol, Itroconauzol, Isavuconazol, Ravuconazol, Posaconazol, Voriconazol, Teraconazol, Abfungin, Terbinafin, Amorolfin, Naftifin, Butenafine, Anidulafungin, Caspofungin, Micafungin, Benzoesäure, Ciclopiroxolamin, Tolnaftat, 5-Fluorocytosin, Griseofulvin, Haloprogin, Fusidinsäure, Gramicidin, Pristinamycin, Ramoplanin, Tyrotricin, Natamycin, Rimocidin, Filipin, Nystatin, Amphotericin B, Candicin, und Hamycin.

Ein weiterer Aspekt der vorliegenden Erfindung betrifft die Verwendung der erfindungsgemäßen Verbindung zur Herstellung eines Medikaments zur Behandlung von Erkrankungen und/oder Schädigungen des Innenohres. Eine Ausführungsform der vorliegenden Erfindung betrifft eine Methode zur Behandlung von Erkrankungen und/oder Schädigungen des Innenohres durch Verabreichung von 6-Fluor-9-methyl-β-carbolin (I).

Ein weiterer Aspekt der vorliegenden Erfindung betrifft pharmazeutische Zusammensetzungen in Form von Tropfen, Salben, Sprays, Liposomen, Gelen, Emulsionen oder Injektionslösungen enthaltend die erfindungsgemäße Verbindung. Ferner betrifft die vorliegende Erfindung pharmazeutische Zusammensetzungen, welche unter Verwendung der erfindungsgemäßen Verbindung oder eines Salzes davon hergestellt wurden.

Neben der Verbindung der vorliegenden Erfindung enthalten die pharmazeutischen Zusammensetzungen bevorzugt einen pharmakologisch verträglichen Träger, Hilfsstoff und/oder Lösungsmittel.

Die pharmazeutischen Zusammensetzungen, die erfindungsgemäße Verbindung enthaltend, können in Form von transdermalen Applikationssystemen (Pflaster, Film), Tropfen, Pillen, Tabletten, Filmtabletten, Schichttabletten, Dragees, Gelen, Hydrogelen, Salben, Sirupen, Granulaten, Suppositorien (Zäpfchen), Emulsionen, Dispersionen, Mikrokapseln, Kapseln, Mikroformulierungen, Nanoformulierungen, Liposomen, Pulvern, Pudern, Sprays, Aerosolen, Lösungen, Säften, Suspensionen, Infusionslösungen oder Injektionslösungen hergestellt und verabreicht werden. Bevorzugt sind pharmazeutische Zusammensetzungen in Form von Liposomen, Gelen und Emulsionen. Insbesondere bevorzugt sind Hydrogelformulierungen.

Derartige Zusammensetzungen sind unter anderem für die Inhalation oder die intravenöse, intraperitoneale, intramuskuläre, subkutane, mucokutane, orale, rektale, transdermale, topikale, buccale, intradermale, intragastrale, intrakutane, intranasale, intrabuccale, perkutane, intratympanale oder sublinguale Verabreichung geeignet. Besonders bevorzugt ist die Verabreichung bzw. Injektion in das Mittelohr sowie die topische Verabreichung auf das Trommelfell.

Als pharmakologisch verträgliche Träger können beispielsweise Lactose, Stärke, Sorbitol, Sucrose, Cellulose, Magnesiumstearat, Dicalciumphosphate, Calciumsulfate, Talk, Mannitol, Ethylalkohol und dergleichen eingesetzt werden. Puder als auch Tabletten können zu 5 bis 95 Gew. % aus einem derartigen Träger bestehen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen. Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkoholen oder denaturierter Gelatine oder Stärke hergestellt. Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, Natrium-Carboxymethylcellulose, mikrokristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Mais, Reis oder Kartoffeln, sowohl natürliche als auch synthetische Gummis wie Akaziengummi oder Guar-Gummi, Gelatine, Tragacanth, Alginsäure, Natriumalginat, Ammonium-Calcium-Alginat, Methylcellulose, Natrium-Carboxymethylcellulose, Hydroxypropyl-methylcellulose, Polyvinylpyrrolidon, Polyethylenglycol, Wachse sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol, Borsäure und Aminosäuren wie Leucin eingesetzt werden. Derartige Gleitmittel können in Mengen von 0,05 bis 15 Gew.-% verwendet werden.

Somit sind alle pharmazeutischen Formulierungen bevorzugt, welche geeignet sind, den Wirkstoff lokal an der Rundfenstermembran zu applizieren. Ferner bevorzugt ist, wenn die pharmazeutischen Formulierungen einen Membranpenetrationsförderer enthalten, der den Durchtritt des 6-Fluor-9-methyl-ß-carbolins durch die Rundfenstermembran unterstützt. Dementsprechend sind flüssige oder gelförmige Formulierungen insbesondere bevorzugt. Natürlich ist es auch möglich, den Wirkstoff oral zu applizieren.

Pharmazeutische Zusammensetzungen für eine beliebige Art der Verabreichung von 6-Fluor-9-methyl-ß-carbolin enthalten eine für den therapeutischen Effekt ausreichende Menge an 6-Fluor-9-methyl-ß-carbolin und, falls nötig, anorganische oder organische, feste oder flüssige pharmazeutisch verträgliche Trägerstoffe. Pharmazeutische Zusammensetzungen, die für eine topische Verabreichung im Mittelohr geeignet sind, beinhalten wässrige Lösungen oder Suspensionen, die vor der Verabreichung im Mittelohr hergestellt werden können, z. B. im Fall von lyophilisierten Formulierungen, die 6-Fluor-9-methyl-ß-carbolin alleine oder zusammen mit einem Träger enthalten.Die pharmazeutischen Zusammensetzungen schließen weiterhin Gele, die biologische abbaubar oder nicht-biologisch abbaubar, wässrig oder nicht-wässrig oder Mikrosphären basiert sind ein. Beispiele für solche Gele beinhalten Polyoxamere, Hyaluronate, Xyloglucane, Chitosane, Polyester, Polylactide, Polyglycolide oder deren Co-Polymer PLGA, Saccharoseacetatisobutyrat und Gylcerinmonooleat. Pharmazeutische Zusammensetzungen, die für eine enterale oder parenterale Verabreichung geeignet sind, beinhalten Tabletten oder Gelatinekapseln oder wässrige Lösungen oder Suspension wie oben beschrieben.

Die pharmazeutischen Zusammensetzungen können sterilisiert werden und/oder Adjuvantien enthalten, z. B. Konservierungsstoffe, Stabilisatoren, Feuchthaltemittel und/oder Emulgatoren, Salze für die Regulierung des osmotischen Drucks und/oder Puffer. Die erfindungsgemäße pharmazeutische Zusammensetzung kann, falls gewünscht, weitere Wirkstoffe enthalten. Diese pharmazeutischen Zusammensetzungen können durch eine beliebige Methode, die aus dem Stand der Technik bekannt ist, hergestellt werden, z. B. durch herkömmliche Methoden wie Vermischung, Granulierung, Konfektionierung, Lösung und Lyophilisierung und können von ungefähr 0,01 bis 100 Gew. - Prozent, bevorzugt zwischen 0,1 und 50 Gew. - Prozent, bei Lyophilisaten bis zu 100 Gew. - Prozent an 6-Fluor-9-methyl-ß-carbolin enthalten.

Bei einer bevorzugten Ausführungsform wird die erfindungsgemäße pharmazeutische Zusammensetzung für eine topische Verabreichung formuliert. Geeignete Träger für eine otogene Verabreichung sind organische oder anorganische Substanzen, die pharmazeutisch verträglich sind und die nicht mit 6-Fluor-9-methyl-ß-carbolin und/oder den weiteren Wirkstoffen reagieren, z. B. Kochsalz, Alkohole, pflanzliche Öle, Benzylalkohole, Alkylglycole, Polyethylenglycole, Glycerintriacetate, Gelatine, Kohlenhydrate wie Lactose oder Stärke, Magnesiumcarbonat (Magnesia, *Chalk*), Stearat (Wachse), Talk und Petrolatum (Vaseline). Die beschriebenen Zusammensetzungen können sterilisiert werden und/oder Hilfsstoffe wie Gleitmittel, Konservierungsstoffe wie Thiomersal (z. B. 50 Gew.-%), Stabilisatoren und/oder Feuchthaltemittel, Emulgatoren, Salze zur Beeinflussung des osmotischen Drucks, Puffersubstanzen, Farbstoffe und/oder Geschmacksstoffe enthalten. Diese Zusammensetzungen können gegebenenfalls auch einen oder mehrere weitere Wirkstoffe enthalten. Erfindungsgemäße otogene Zusammensetzungen können verschiedene Stoffe und/oder Substanzen umfassen, z.B. andere biologisch aktive Substanzen wie Antibiotika, antiinflammatorische Wirkstoffe wie Steroide, Cortisone, Analgetika, Antipyrine, Benzocaine, Procaine. Erfindungsgemäße Zusammensetzungen für die topische Verabreichung können andere pharmazeutische verträgliche Stoffe und/oder Substanzen enthalten. In bevorzugten Ausführungsformen der vorliegenden Erfindung wird ein topischer Exzipient ausgewählt, der die Abgabe von 6-Fluor-9-methyl-ß-carbolin und des gegebenenfalls weiteren Wirkstoffs oder der Wirkstoffe an das Blutkreislaufsystem oder an das Zentralnervensystem nicht verstärkt, wenn dieser am Ohr, im Mittelohr oder im Gehörgang verabreicht wird. Mögliche Trägerstoffe beinhalten Hydrocarbonsäuren, wasserfreie Absorptionsmittel wie hydrophiles Petrolatum (Vaseline) und wasserfreies Lanolin (z. B. Aquaphor^{®}) und Mittel auf Basis von Wasser-Öl-Emulsionen wie Lanolin und *Cold Cream.* Mehr bevorzugt sind Trägerstoffe, die im wesentlichen nicht-ausschließend sind und beinhalten üblicherweise diejenigen Trägerstoffe, die wasserlöslich sind, wie auch Stoffe auf Basis von Öl-in-Wasser-Emulsionen (Cremes oder hydrophile Salben) und Stoffe auf wasserlöslicher Basis wie auf Polyethylenglycol basierende Trägerstoffe und wässrige Lösungen, die mit verschiedenen Stoffen wie Methylcellulose, Hydroxyethylcellulose und Hydroxpropylmethylcellulose geliert wurden.

Bei oraler Verabreichung in Form von Tabletten oder Kapseln kann das erfindungsgemäße 6-Fluor-9-methyl-ß-carbolin mit nicht-toxischen pharmazeutisch verträglichen Hilfsstoffen ausgewählt aus der Liste umfassend Bindemittel wie vorgelierte Maisstärke, Polyvinylpyrrolidon oder Hydroxypropylmethylcellulose; Füllstoffe wie Lactose, Saccharose, Glucose, Mannitol, Sorbitol und andere reduzierende und nicht-reduzierende Zucker, mikrokristalline Cellulose, Calciumsulfat oder Calciumhydrogenphosphat; Gleitmittel wie Magnesiumstearat, Talkum oder Silica, Stearinsäure, Natriumstearylfumarat, Glycerylbehenat, Calciumstearat und ähnliche; Zerfallsbeschleuniger wie Kartoffelstärke oder Natriumglycolat-Stärke; Befeuchtungsmittel wie Natriumdodecylsulfat; Farbstoffe; Geschmacksstoffe; Gelatine; Süßstoffe; natürliche und syntheische Gummis wie Gummi arabicum, Tragacanth oder Alginate; Puffersalze; Carboxymethylcellulose; Polyethylenglycol; Wachse und ähnliche.

Die Tabletten können mit einer konzentrierten Zuckerlösung umhüllt sein, die zum Beispiel Gummi arabicum, Gelatine, Talcum, Titandioxid und ähnliches enthalten kann. Bei einer anderen Ausführungsform können die Tabletten mit einem Polymer umhüllt sein, das sich in einem leichtflüchtigen organischen Lösungsmittel oder einer Mixtur an organischen Lösungsmitteln löst. In bevorzugten Ausführungsformen ist das erfindungsgemäße 6-Fluor-9-methyl-ß-carbolin als Tablette zur sofortigen Freigabe oder als Tablette in Retardform formuliert. Dosierungsformen zur sofortigen Freigabe erlauben die Freisetzung eines Großteils oder der Gesamtmenge an 6-Fluor-9-methyl-ß-carbolin innerhalb einer kurzen Zeitspanne wie 60 Minuten oder weniger und ermöglichen die rasche Absorption des 6-Fluor-9-methyl-ß-carbolins. Retardformen erlauben die verzögerte Freisetzung über einen längeren Zeitraum, um einen therapeutisch wirksamen Plasmaspiegel an 6-Fluor-9-methyl-ß-carbolin zu erreichen und/oder diesen therapeutisch wirksamen Plasmaspiegel über einen längeren Zeitraum stabil zu halten und/oder andere pharmakokinetische Eigenschaften des 6-Fluor-9-methyl-ß-carbolins zu modifizieren.

Zur Formulierung von Weichgelatinekapseln kann das erfindungsgemäße 6-Fluor-9-methyl-ß-carbolin zum Beispiel mit einem pflanzlichen Öl oder Polyethylenglycol vermengt werden. Hartgelatinekapseln können das 6-Fluor-9-methyl-ß-carbolin in Granulatform enthalten unter Verwendung von entweder den oben genannten Hilfsstoffen für Tabletten wie Lactose, Saccharose, Mannitol, Stärken wie Kartoffelstärke, Maisstärke oder Amylopectin, Cellulosederivate oder Gelatine. Auch flüssige oder semiflüssige Formen des erfindungsgemäßen 6-Fluor-9-methyl-ß-carbolins können in die Hartgelatinekapseln gefüllt werden.

Das erfindungsgemäße 6-Fluor-9-methyl-ß-carbolin kann auch in Mikrokügelchen oder Mikrokapseln, die z. B. aus Polyglycolsäure/Milchsäure (PGLA) hergestellt sind, eingebracht werden. Bioverträgliche Polymere, ausgewählt aus der Liste umfassend Polymilchsäure, Polyglycolsäure, Copolymere von Polymilchsäure und Polyglycolsäure, Poly-ε-Caprolacton, Polyhydroxybuttersäure, Polyoethoester, Polyacetale, Polyhydropurane, Polycyanoacrylate und vernetzte oder amphipathische Blockcopolymere von Hydrogelen, können verwendet werden, um eine kontrollierte Freisetzung des erfindungsgemäßen 6-Fluor-9-methyl-ß-carbolins aus den pharmazeutischen Zusammensetzungen der vorliegenden Erfindung zu erreichen.

In einer weiteren Ausführungsform der Erfindung wird das 6-Fluor-9-methyl-ß-carbolin als eine flüssige Formulierung zur oralen Verabreichung bereitgestellt. Flüssige Zubereitungen für die orale Verabreichung können in Form von Lösungen, Sirupen, Emulsionen oder Suspensionen bereitgestellt werden. Alternativ können die flüssigen Formulierungen zur oralen Verabreichung durch Rekonstitution der trockenen Formulierung zur oralen Verabreichung mit Wasser oder einem anderen geeignetem Trägerstoff vor der Anwendung hergestellt werden. Zubereitungen zur oralen Verabreichung können so formuliert werden, dass eine kontrollierte oder retardierte Freisetzung des erfindungsgemäßen 6-Fluor-9-methyl-ß-carbolins und gegebenenfalls weiterer Wirkstoffe erreicht werden kann.

Bei oraler Verabreichung in flüssiger Form kann das erfindungsgemäße 6-Fluor-9-methyl-ß-carbolin mit nicht-toxischen pharmazeutisch verträglichen inerten Trägerstoffen wie Ethanol, Glycerin, Wasser; Suspensionsmitteln wie Sorbitolsyrup, Cellulosederivate oder gehärteten essbaren Fetten; Emulgatoren wie Lecithin oder Gummi arabicum; nicht-wässrigen Trägerstoffen wie Mandelöl, öligen Estern, Ethanol oder fraktionierten pflanzlichen Ölen; Konservierungsstoffen wie Methyl-oder Propyl-p-hydroxybenzoaten oder Sorbinsäure; und ähnlichen. Stabilisatoren wie z. B. Antioxidantien wie Butylhydroxyanisol, Butylhydroxytoluol, Propylgallat, Natriumascorbat, Zitronensäure können auch zur Stabilisierung der Dosierungsform verwendet werden. Zum Beispiel können die Lösungen ungefähr 0,2 Gew.-% bis ungefähr 20 Gew.-% an 6-Fluor-9-methyl-ß-carbolin enthalten, wobei die Ausgleichsmasse Zucker und eine Mischung aus Ethanol, Wasser, Glycerin und Propylenglycol sein kann. Optional können diese flüssigen Formulierungen Farbstoffe, Geschmacksstoffe, Saccharin, Carboxylcellulose als Verdickungsmittel und/oder andere Hilfsstoffe enthalten.

Bei einer weiteren Ausführungsform wird eine therapeutisch wirksame Dosis an erfindungsgemäßem 6-Fluor-9-methyl-ß-carbolin über eine Lösung oral verabreicht, wobei die Lösung einen Konservierungsstoff, Süßstoff, Lösungsvermittler und ein Lösungsmittel enthalten kann. Die Lösung zur oralen Verabreichung kann einen oder mehrere Puffer, Geschmackstoffe oder weitere Exzipienten enthalten. Bei einer weiteren Ausführungsform wird Pfefferminze oder ein anderer Geschmacksstoff zu der Lösung des erfindungsgemäßen 6-Fluor-9-methyl-ß-carbolins für die orale Verabreichung hinzugefügt.

Für die Verabreichung über Inhalierung kann das erfindungsgemäße 6-Fluor-9-methyl-ß-carbolin in geeigneter Weise verabreicht werden, wie in der Darreichungsform als Aerosolspray mit unter Druck stehenden Packungen oder einem Zerstäuber, unter Verwendung eines geeigneten Treibgases wie Dichlorfluormethan, Trichlorfluormethan, Dichlortetrafluorethan, Kohlendioxid oder eines anderen geeigneten Gases. Bei Verwendung eines unter Druck stehenden Aerosols kann die Dosis dadurch bestimmt werden, dass ein Ventil bereitgestellt wird, um eine abgemessene Menge zu verabreichen. Kapseln und Kartuschen aus z. B. Gelatine für die Verwendung in Inhalatoren oder Insufflatoren können so formuliert werden, dass die Kapseln und Kartuschen eine Pulvermischung des erfindungsgemäßen 6-Fluor-9-methyl-ß-carbolins und gegebenenfalls eines weiteren oder mehrerer weiterer Wirkstoffe sowie eine geeignete Pulverbasissubstanz wie Lactose oder Stärke enthalten.

Lösungen für die parenterale Verabreichung durch Injektion können aus einer wässrigen Lösung eines wasserlöslichen pharmazeutisch verträglichen Salzes des erfindungsgemäßen 6-Fluor-9-methyl-ß-carbolins mit einer Konzentration von ungefähr 0,5 Gew.-% bis ungefähr 10 Gew.-% hergestellt werden. Diese Lösungen können auch Stabilisatoren und/oder Puffersubstanzen enthalten und können in geeigneter Weise über Ampullen mit verschiedenen Dosiseinheiten bereitgestellt werden.

Somit ist die hier vorgestellte erfindungsgemäße Verbindung nützlich für die Herstellung von pharmazeutischen Zusammensetzungen zur Behandlung von Erkrankungen und/oder Schädigungen des Innenohrs. Hierbei handelt es sich bei den Erkrankungen und/oder Schädigungen des Innenohrs bevorzugt um akute und/oder chronische Erkrankungen des Innenohrs. Zu den akuten Erkrankungen des Innenohrs gehören unter anderem Knalltrauma, Explosionstrauma des Ohres, Hörsturz und Insertionstrauma. Zu den chronischen Erkrankungen des Innenohrs gehören unter anderem chronisches Schalltrauma, Tinnitus und Presbyakusis. Die Erkrankungen und/oder Schädigungen des Innenohrs können zudem dadurch gekennzeichnet sein, dass diese durch ototoxische Substanzen bedingt sein können.

### Beschreibung der Figuren

- Figur 1: Reaktionsschema zur dreistufigen Reaktion von 5-Fluor-1-methyltryptamin Hydrochlorid (II) und Glyoxalsäurehydrat (III) über das Zwischenprodukt (IV) und 6-Fluor-9-methyl-2,3,4,9-tetrahydro-ß-carbolin (V) zu 6-Fluor-9-methyl-ß-carbolin (I).
- Figur 2:: SH-SY5Y-Zelllinie 15 Tage Inkubation mit AC102
- Figur 3:: SH-SY5Y-Zelllinie 15 Tage Inkubation mit AC002

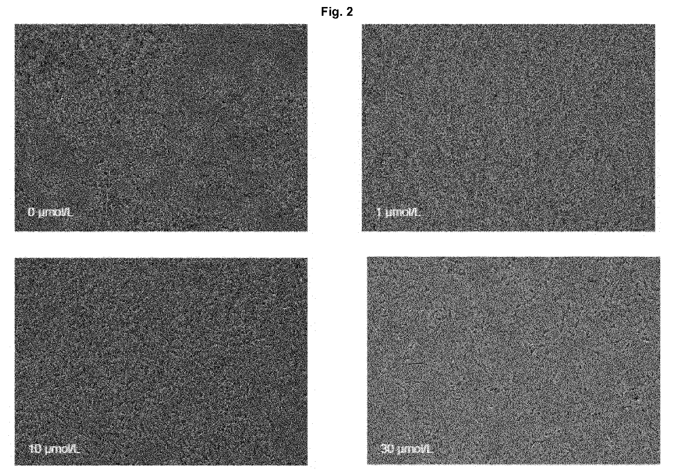

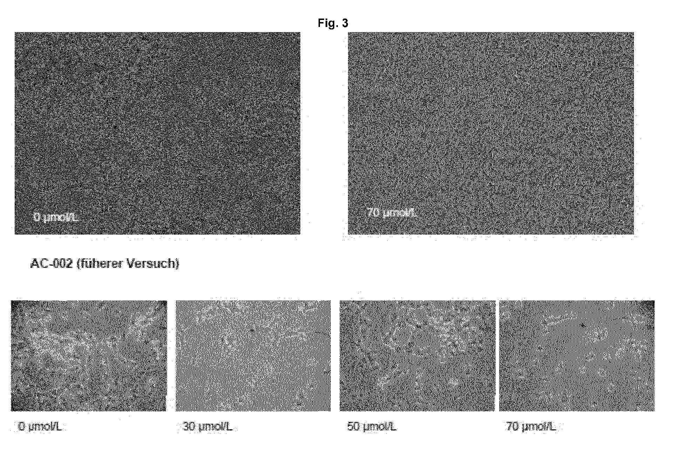

- Figur 2 und 3: zeigen die Kultur von humanen Neuroblastomzellen (SH-SY5Y), die mit ansteigenden Konzentrationen von 6-Fluor-9-methyl-ß-carbolin (AC102) 15 Tage lang exponiert wurden (Fig. 2). Zum Vergleich ist in der Fig. 3 unter denselben Bedingungen die Wirkung von 9-Methyl-ß-Carbolin (AC002) dargestellt. Die obere Reihe stammt aus dem aktuellen Experiment mit der Konzentration 70 µmol/L, während die untere Reihe die Konzentrationsabhängigkeit aus einem früheren Experiment zeigt. Die erfindungsgemäße Verbindung (AC102) hemmt die Proliferation der Zellen und induziert die Aussprossung von schlanken Fortsätzen sowie eine Veränderung von Größe und Form der Zellen. Dies ist mit der Induktion einer Differenzierung zu erklären. Besonders deutlich wird diese Wirkung der Verbindung beim Vergleich der Konzentrationen 0 µM (Kontrolle) und 30 µM zu beiden Zeitpunkten.

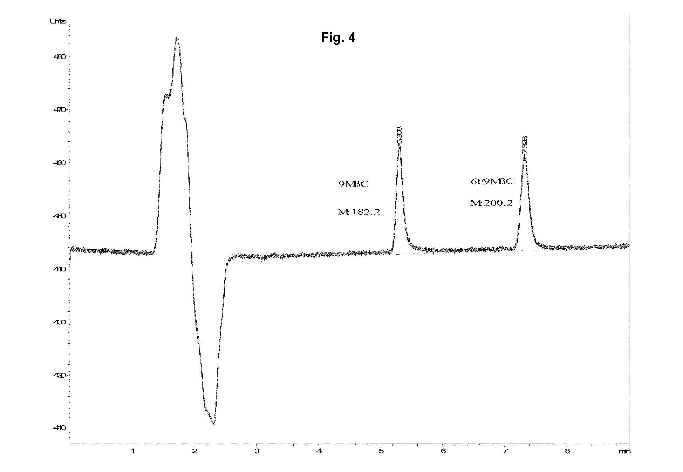

- Figur 4: HPLC Chromatogramm von 9-Methyl-β-carbolin (9MßC, AC002) und 6-Fluor-9-methyl-β-carbolin (6F9MßC, AC102). Die Retentionszeiten betrugen 5,3 bzw. 7,3 Min. Säule: Phenomenex Luna 3uC18 (2) 100A; Eluent: ACN90%/NH₄Ac 40mM [45/55]

### Beispiele

### Beispiel 1: Synthese von 6-Fluor-9-methyl-β-carbolin

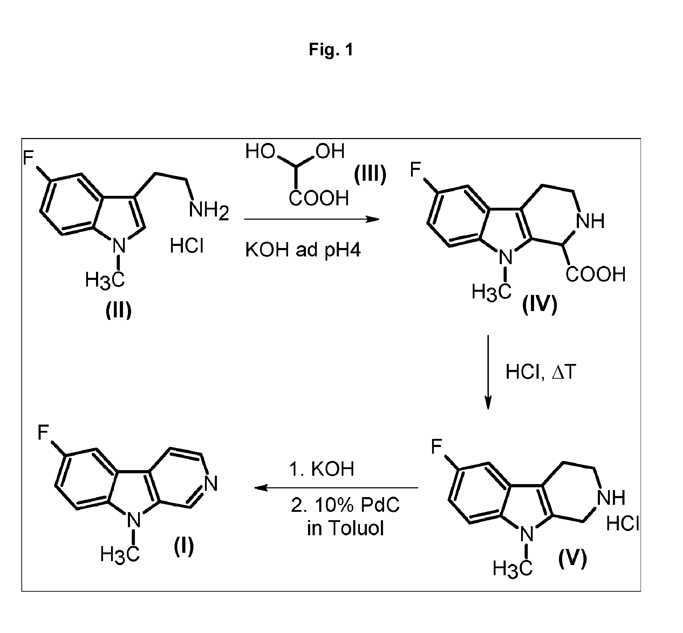

Analog zur Vorschrift von Ho und Walker (Beng T. Ho und K. E. Walker; Org. Synth. 1971, 51, 136 oder Org. Synth. 1988, Coll. Vol. 6, 965) werden in 10 mL Wasser 3,5 mmol (800 mg) 5-Fluor-1-methyltryptamin Hydrochlorid **(****Figur 1****, Formel II,** AKos GmbH, Steinen) gelöst und mit einer Lösung von 3,9 mmol (356,4 mg) Glyoxalsäurehydrat **(****Figur 1****, Formel III)** in 810 µL Wasser vereinigt. Unter Rühren wird dann eine Lösung aus 3,4 mmol (190,4 mg) Kaliumhydroxid hinzupipettiert, wobei der pH auf etwa 4 eingestellt wird. Die Reaktionsmischung wird 3 h bei Raumtemperatur gerührt und zur Vervollständigung der Kristallisation noch eine weitere Stunde ins Eisbad gestellt. Der orange-beige Niederschlag des Betains **(****Figur 1****, Formel IV)** wird abfiltriert und mit wenig Eiswasser gewaschen.
Zur Decarboxylierung wird der noch feuchte Filterkuchen des Betains **(****Figur 1****, Formel IV)** in einen Kolben überführt und in verdünnter Salzsäure (6,48 mLWasser und 918 µL konzentrierte Salzsäure) gelöst. Die Reaktionsmischung wird unter Stickstoff 10 min refluxiert, nochmals 945 µL konzentrierte Salzsäure über ein Septum zugegeben und nochmals 15 min refluxiert. Anschließend wird die Lösung wieder auf Raumtemperatur gebracht und 12 h bei 0-5 °C gelagert. Die entstehenden Kristalle des 6-Fluor-9-methyl-2,3,4,9-tetrahydro-ß-carbolins **(****Figur 1****, Formel V)** werden abfiltriert und aus Methanol umkristallisiert. Ausbeute: 230 mg (52 %).
Zur Aromatisierung werden die 230 mg 6-Fluor-9-methyl-2,3,4,9-tetrahydro-ß-carbolin **(****Figur 1****, Formel V)** in 15 mL Wasser gelöst, mit Natriumhydroxid auf pH 13 gebracht und mit einer Mischung aus Ethylacetat und Toluol (1:1) 3 x extrahiert. Die vereinigten organischen Phasen werden mit Magnesiumsulfat (MgSO₄) getrocknet und unter reduziertem Druck eingedampft. Der ölige Rückstand wird mit 50 mL Toluol aufgenommen, mit 100 mg Pd/C (10 %) versetzt und 24 h refluxiert. Von der noch warmen Lösung wird Pd/C abfiltriert und der Filterkuchen mit 20 mL warmem Toluol gewaschen. Die organische Phase wird im Vakuum abgedampft. Der ölige Rückstand aus 6-Fluor-9-methyl-β-carbolin **(****Figur 1****, Formel I)** kristallisiert beim Stehen aus und bildet 183 mg (96 %) senfgelbe Kristalle.

Schmelzpunkt: 117-118 °C; GC/MS für die freie Base: m/z = 201 (10 %), 200 (100 %), 199 (71 %), 185 (13 %), 145 (6 %), 131 (6 %), ¹H-NMR: δ (ppm) Methanol d4, 400 MHz: 3,80, s, 3H, N-CH₃, 7,34, m, ¹H; 7,43, m, ¹H; 7,73, m, ¹H; 7,91, m, ¹H; 8,24, m, ¹H; 8,73, m, ¹H, ¹³C-NMR: δ (ppm) Methanol d4, 100 MHz: 28,23; 106,38; 106,64; 110,20 and 110,29, d; 114,67; 116,25; 116,52; 120,59 and 120,66, d; 127,91; 131,30; 136,95; 137,51; 138,35; 156,20 and 158,55, d,
Elementaranalyse: für C₁₂H₉FN₂ x 0,1 H₂O x 0,1 Toluol: C, H, N,
Berechnet: C: 72,21; H: 4,77; N: 13,26, Gefunden: C: 72,01; H: 4,55; N: 13,45

### Beispiel 2: Differenzierung von humanen Neuroblastomzellen (SH-SY5Y)

Die SH-SY5Y-Zelllinie wurde von der deutschen Sammlung von Mikroorganismen und Zellkulturen - DSMZ, Braunschweig bezogen. Die Zellen wurden in MEM (Minimum essential medium, Life Technologies) mit 10 % FCS (fetales Kälberserum, Biochrom AG) und Penicillin/Streptomycin (Biochrom AG) kultiviert. Für die morphologischen Untersuchungen wurden die Zellen in einer Dichte von 1,5 x 10⁴ Zellen/cm² in 24-Wellplatten (Falcon, cell culture treated) mit 1 mL Medium ausgesät. Eine Stammlösung von 10 mmol/L der erfindungsgemäßen Verbindung wurde in 50 % Ethanol hergestellt und steril filtriert. Fünf Stunden nach dem Aussäen der Zellen wurde die erfindungsgemäße Verbindung in der entsprechenden Menge zum Medium gegeben, um die gewünschte Endkonzentration zu erhalten. Das Medium wurde einmal pro Woche gewechselt. Die Zellen wurden regelmäßig mikroskopisch begutachtet. Mikroskopische Aufnahmen wurden am BZ Keyence 10 mit einem 10 x Objektiv und 4 x Digitalzoom aufgenommen. Eine automatische Kontrastverstärkung wurde mit dem Programm BZ Analyzer vorgenommen.
Die Befunde ergeben einen Vorteil der erfindungsgemäßen Verbindung gegenüber 9-Methyl-ß-carbolin, da eine vergleichbare Differenzierung der getesteten Zellen erst bei einer Konzentration von etwa 70 µM 9-Methyl-ß-carbolin im Vergleich zu 30 µM 6-Fluor-9-Methyl-ß-carbolin beobachtet wird. 50 µM 9-Methyl-ß-carbolin führten zu einem geringeren Neuritenwachstum, als Anzeichen einer Differenzierung der humanen Neuroblastomzellen, als 30 µM der erfindungsgemäßen Verbindung.

### Beispiel 3: Lipophilie von 6-Fluor-9-methyl-ß-carbolin im Vergleich zu 9-Methyl-ß-carbolin

Die folgenden Experimente wurden durchgeführt um zu prüfen, ob die Lipophilie von 6-Fluor-9-methyl-ß-carbolin größer ist als die des 9-Methyl-ß-carbolins. Experimentell wird die Lipophilie entweder durch die tatsächliche Verteilung zwischen Octanol und Wasser bestimmt, oder einfacher, wie von Valkó beschrieben, durch HPLC-Experimente mit einer Umkehrphasen-Säule (RP18) (K. Valkó; Journal of Chromatography A 2004,1037 (1-2), 299-310).. Hierbei wechselwirken die Bestandteile der zu untersuchenden Substanz unterschiedlich stark mit der stationären Phase. Wechselwirkt ein Bestandteil schwächer mit der stationären Phase, verlässt er die Säule früher. Je nach Stärke dieser Wechselwirkungen erscheinen die Bestandteile der Substanz zu verschiedenen Zeiten (den Retentionszeiten) am Ende der Trennsäule. Solche Experimente wurden für die beiden Substanzen durchgeführt. Es zeigte sich, dass die erfindungsgemäße Verbindung 6-Fluor-9-methyl-ß-carbolin eine längere Retentionszeit hat als 9-Methyl-ß-carbolin (Figur 4), was bei der ausgewählten Säule bzw. stationären Phase eindeutig auf eine höhere Lipophilie des 6-Fluor-9-methyl-ß-carbolins hinweist.

Zudem wurde die Löslichkeit beider Verbindungen in Wasser bestimmt, indem für beide Verbindungen eine Konzentrationsreihe in Wasser bei Raumtemperatur angesetzt wurde. Die Konzentration, die noch gerade löslich war, wurde durch eine externe Kalibrierung bestimmt. Die entsprechende Konzentration wurde mittels HPLC quantifiziert. Es wurde eine maximal lösliche Konzentration des 6-Fluor-9-methyl-ß-carbolins (Base) von 29,7 mg/L und des 9-Methyl-ß-carbolins (Base) von 168,3 mg/L gefunden. Diese geringere Wasserlöslichkeit des 6-Fluor-9-methyl-ß-carbolins stützt im Umkehrschluss die erhöhte Lipophilie im Vergleich zu 9-Methyl-ß-carbolin.

Die Lipophilie eines Stoffes hat einen starken Effekt auf seine ADME Parameter ("absorption", "distribution", "metabolism", und "excretion"). Durch die gesteigerte Lipophilie werden Stoffe vor allem in lipophilen Kompartimenten angereichert. Um Membranen passieren zu können, müssen chemische Stoffe den hydrophoben Bereich einer biologischen Doppelmembran passieren können, sie müssen also selbst lipophil sein. Oft besteht eine gute Korrelation zwischen Lipidlöslichkeit und Membranpassage. Somit beeinflusst die Lipophilie ganz entscheidend die Pharmakokinetik einer Verbindung. Zusammengefasst zeigen mehrere Experimente eine deutliche höhere Lipophilie von 6-Fluor-9-methyl-ß-carbolin gegenüber 9-Methyl-ß-carbolin

Ein weiterer wichtiger Aspekt der erfindungsgemäßen Verbindung betrifft die Verlängerung der Eliminationshalbwertszeit. Während das 9-Methyl-ß-carbolin über eine Hydroxylierung an Position 6 mit anschließender Konjugationsreaktion abgebaut wird, ist dieser Metabolisierungsweg beim 6-Fluor-9-methyl-ß-carbolin durch die Fluoridgruppe blockiert. Wahrscheinlich ist dies der Grund für eine Erhöhung der Halbwertszeit des 6-Fluor-9-methyl-ß-carbolins verglichen mit 9-Methyl-ß-carbolin. Da die Halbwertszeit von Harman, einem Strukturanalogon von 9-Methyl-ß-carbolin, im Blutplasma des Menschen etwa 68 Min beträgt (Rommelspacher et al.; Eu J Pharmacol 2002, 441 (1-2), 115-125). , stellt eine Verlängerung dieses Werts einen relevanten Vorteil der erfindungsgemäßen Verbindung dar, insbesondere bei Anwendungen zur Behandlung von chronischen Erkrankungen.

Literatur:
1.) Application of high-performance liquid chromatography based measurements of lipophilicity to model biological distribution. Valkó K Journal of Chromatography A 2004; 1037 (1-2): 299-310.
2.) The levels of norharman are high enough after smoking to affect monoamineoxidase B in platelets. Rommelspacher et al., Eu J Pharmacol 2002;441 (1-2): 115-125.

## Patentansprüche

1. Verbindung 6-Fluor-9-methyl-β-carbolin der Formel (I) 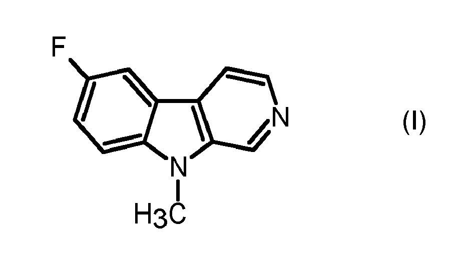 sowie pharmakologisch verträgliche Salze, Solvate und Hydrate der vorgenannten Verbindung.

2. Verfahren zur Herstellung der Verbindung gemäß Anspruch 1, umfassend die folgenden Schritte:
a) Reaktion der Ausgangsverbindung 5-Fluor-1-methyltryptaminhydrochlorid der Formel (II) unter Zugabe von Glyoxalsäurehydrat der Formel (III) und einer Base zur Verbindung der Formel (IV) 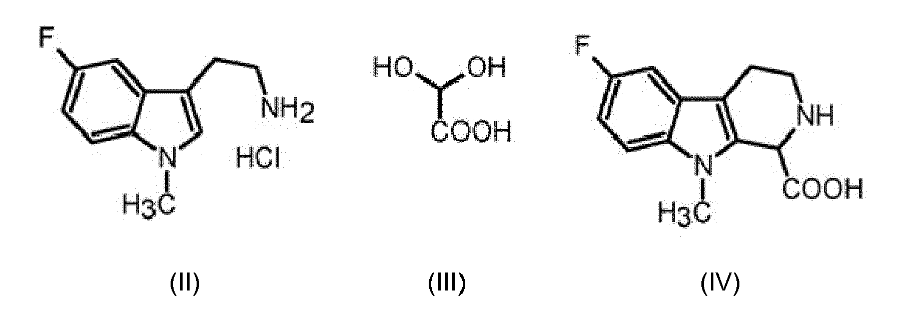
b) Decarboxylierung der Verbindung der Formel (IV) unter Zugabe einer Säure und unter Erhitzen zu 6-Fluor-9-methyl-2,3,4,9-tetrahydro-ß-carbolin der Formel (V) 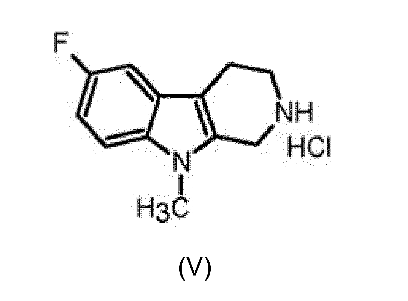
c) Aromatisierung von 6-Fluor-9-methyl-2,3,4,9-tetrahydro-ß-carbolin (V) zu 6-Fluor-9-methyl-β-carbolin (I) unter Zugabe eines Katalysators.

3. Verfahren gemäß Anspruch 2, **dadurch gekennzeichnet, dass** es sich bei dem in Schritt c) verwendeten Katalysator um Pd / C handelt.

4. Die Verbindung gemäß Anspruch 1 zur Verwendung als Medikament.

5. Die Verbindung gemäß Anspruch 1 zur Behandlung von Erkrankungen und/oder Schädigungen des Innenohres.

6. Die Verbindung gemäß Anspruch 5, **dadurch gekennzeichnet, dass** es sich bei den Erkrankungen und/oder Schädigungen des Innenohres um akute Erkrankungen des Innenohres handelt, ausgewählt aus der Gruppe umfassend Knalltrauma, Explosionstrauma des Ohres, Hörsturz und Insertionstrauma.

7. Die Verbindung gemäß Anspruch 5, **dadurch gekennzeichnet, dass** es sich bei den Erkrankungen und/oder Schädigungen des Innenohres um chronische Erkrankungen des Innenohres handelt, ausgewählt aus der Gruppe umfassend chronisches Schalltrauma, Tinnitus und Presbyakusis.

8. Die Verbindung gemäß einem der Ansprüche 5 bis 7, **dadurch gekennzeichnet, dass** die Schädigung des Innenohrs durch ototoxische Substanzen bedingt ist.

9. Pharmazeutische Zusammensetzung in Form von Tropfen, Salben, Sprays, Liposomen, Gelen, Emulsionen oder Injektionslösungen enthaltend die Verbindung gemäß Anspruch 1.
